(19) Europäisches Patentamt
European Patent Office
Office européen des brevets

(11) Numéro de publication: **0 055 197**
**B1**

(12) **FASCICULE DE BREVET EUROPEEN**

(45) Date de publication du fascicule du brevet: **04.04.84**

(51) Int. Cl.³: **C 07 C 37/00**, C 07 C 39/27

(21) Numéro de dépôt: **81420186.9**

(22) Date de dépôt: **18.12.81**

(54) **Procédé de préparation de phénols métachlorés.**

(30) Priorité: **24.12.80 FR 8027937**

(43) Date de publication de la demande: **30.06.82 Bulletin 82/26**

(45) Mention de la délivrance du brevet: **04.04.84 Bulletin 84/14**

(84) Etats contractants désignés: **BE DE FR GB IT LU NL**

(56) Documents cités:
**DE - C - 432 802**
**FR - A - 2 209 738**
**FR - A - 2 285 355**

**CHEMICAL ABSTRACTS, vol. 86, no. 7, 14 février 1977, ref. no. 43346k, page 503 COLUMBUS OHIO (US)**

(73) Titulaire: **RHONE-POULENC SPECIALITES CHIMIQUES, "Les Miroirs" 18, Avenue d'Alsace, F-92400 Courbevoie (FR)**

(72) Inventeur: **Cordier, Georges, 50, Chemin des Hermières, F-69340 Francheville (FR)**

(74) Mandataire: **Vignally, Noel et al, RHONE-POULENC RECHERCHES Centre de recherches de Saint Fons Service Brevets Boîte postale 62, F-69190 Saint Fons (FR)**

**EP 0 055 197 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Procede de preparation de phenols metachlores

La présente invention a pour objet un procédé de préparation de phénols comportant un atome de chlore sur l'une au moins des positions méta par rapport à l'hydroxyle phénolique, par hydrodéchloration de chlorophénols plus fortement chlorés.

Par la suite, pour des raisons de commodité, on désignera par l'expression »phénols métachlorés« les phénols comportant un atome de chlore sur l'une au moins des positions méta.

Les phénols métachlorés, et en particulier le chloro-3 phénol et le dichloro-3,5 phénol, sont des composés qui revêtent un très grand intérêt industriel comme intermédiaires en synthèse organique.

Diverses méthodes de préparation des phénols métachlorés ont été proposées. On distingue notamment des méthodes engendrant la fonction phénol sur des composés aromatiques chlorés (par exemple l'hydrolyse alcaline des polychlorobenzènes; la nitration des chloro-3 et dichloro-3,5 benzènes suivie de la réduction du groupe nitro en groupe amino, de la diazotation de ce dernier et de la décomposition du sel de diazonium); des méthodes de chloration de phénols et des méthodes de déchloration de polychlorophénols. Cette dernière méthode présente un très grand intérêt industriel en raison de la disponibilité des polychlorophénols dont certains sont des produits courants et d'autres des sous-produits d'intérêt limité qu'il importe de valoriser.

C'est ainsi, par exemple, que l'on obtient des tri-et tétrachlorophénols isomères dont certains comportent un ou deux atomes de chlore en position méta par rapport à l'hydroxyle phénolique lors de la préparation du tétrachloro-2,3,4,6 phénol et du pentachlorophénol par chloration du dichloro-2,6 phénol sous-produit de la préparation du dichloro-2,4 phénol. Ces divers polychlorophénols constituent des matières premières de choix pour la préparation des phénols métachlorés par déchloration. Une méthode d'élimination des atomes de chlore excédentaires consiste à soumettre les polychlorophénols à une hydrogénation en phase vapeur ou en phase liquide en présence d'un catalysateur. Pour des raisons de simplification, on désignera par la suite par »hydrodéchloration« la déchloration des polychlorophénols par hydrogénation.

Le problème essentiel posé par l'hydrodéchloration des polychlorophénols en chloro-3 ou dichloro-3,5 phénols réside dans l'élimination sélective des atomes de chlore en position 2 et/ou 4 et/ou 6 par rapport à l'hydroxyle phénolique. On a proposé divers procédés d'hydrodéchloration des polychlorophénols mais aucun ne s'est révélé jusqu'ici pleinement satisfaisant.

Ainsi, dans le brevet américain 2 803 669 délivré le 20 août 1957 on a décrit un procédé d'hydrodéchloration de polychlorophénols en phase vapeur par passage d'un mélange gazeux hydrogène/polychlorophénols sur un catalyseur à base d'halogénures cuivreux (chlorure cuivreux par exemple) déposés sur de l'alumine, maintenu à température élevée (350 à 550°C). Appliqué à l'hydrodéchloration du tétrachloro-2,3,4,6 phénol, ce procédé n'a pas permis d'obtenir une élimination sélective des atomes de chlore en position 2,4 et 6 par rapport à l'hydroxyle phénolique. La masse réactionelle résultant de l'hydrogénation est constituée pour l'essentiel de dichloro-2,4 et de dichloro-2,6 phénols.

Dans la demande de brevet français No. 73-43.484 publiée sous le No. 2 209 738, on a proposé un procédé de préparation de phénols métahalogénés par déshalogénation des polyhalogénophénols par hydrogénation en phase liquide à température élevée, en présence d'un catalyseur comportant soit un ou plusieurs sulfures ou polysulfures de fer, de nickel ou de cobalt, soit un métal noble tel que le palladium ou le platine associé à un dérivé du soufre. La réaction est de préférence conduite en présence d'une base telle que les hydroxydes ou les carbonates alcalins pour neutraliser les hydracides engendrés par la réaction au fur et à mesure de leur formation. Bien que ce procédé se montre très sélectif vis-à-vis de la formation des phénols métachlorés, il présente l'inconvénient de devoir être mis en œuvre en présence d'une base et en particulier d'une base alcaline dans des conditions de température (cette dernière doit être comprise de préférence entre 180 et 330°C) favorable à la formation d'halogénodioxines et en particulier de polychlorodioxines dont on sait que certaines présentent une toxicité élevée. Un tel inconvénient enlève en pratique tout intérêt industriel à ce procédé. En définitive l'industrie est encore à la recherche d'un procédé sélectif d'obtention de phénols métachlorés par hydrodéchloration de polychlorophénols s'affranchissant de la présence de bases alcalines. La présente invention se propose précisément de mettre à la disposition de l'industrie un procédé d'hydrodéchloration sélective des polychlorophénols exempt des inconvénients précités.

Plus spécifiquement, la présente invention a pour objet un procédé d'obtention sélective de chlorophénols comportant un atome de chlore sur l'une au moins des positions méta par rapport à l'hydroxyle phénolique, par hydrogénation à chaud en phase liquide, en présence d'un catalyseur à base d'un métal noble du groupe VIII de la classification périodique, de polychlorophénols de formule générale (I):

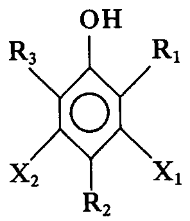

(I)

dans laquelle:

— $X_1$ et $X_2$, identiques ou différents représentent un atome de chlore, un atome d'hydrogène, un radical alkyle, aryle, arylalkyle, alkoxy, aryloxy, l'un au moins des symboles $X_1$ et $X_2$ représentant un atome de chlore,
— $R_1$, $R_2$, et $R_3$, identiques ou différents, représentent un atome de chlore, un atome d'hydrogène, un radical alkyle, un radical aryle, arylalkyle, un radical alkoxy, aryloxy, l'un au moins des symboles $R_1$, $R_2$, et $R_3$ représentant un atome de chlore,

caractérisé en ce que la réaction est conduite dans un milieu solvant acide, au moins partiellement aqueux, en présence d'au moins un métal lourd appartenant aux groupes 1b, 2b, 3a, 4a et 5a de la classification périodique des éléments (cf. HANDBOOK OF CHEMISTRY AND PHYSICS édité par R. C. WEAST, 53ème édition 1972—1973).

Dans la formule (I), ceux des radicaux $X_1$, $X_2$, $R_1$ et $R_3$ qui ne symbolisent pas un atome de chlore représentent plus spécifiquement un radical alkyle comportant de 1 à 10 atomes de carbone et de préférence de 1 à 4 atomes de carbone comme les radicaux méthyle, éthyle, n-propyle, isopropyle, n-butyle, isobutyle, t-butyle; un radical phényle; un radical benzyle; un radical alkoxy comportant de 1 à 10, et de préférence de 1 à 4 atomes de carbone comme les radicaux méthoxy, éthoxy, n-propyloxy, isopropyloxy, n-butyloxy; le radical phénoxy.

Le milieu solvant dans lequel est effectuée la réaction peut être constitué par l'eau seule ou par un mélange en toutes proportions d'eau et d'un ou plusieurs solvants organiques, liquides et inertes dans les conditions de la réaction. Il n'est pas nécessaire que ce ou ces solvants soient miscibles à l'eau; leur rôle consiste essentiellement à dissoudre les polychlorophénols. Comme exemples de solvants on peut citer: des hydrocarbures aliphatiques tels que l'ocrane, l'hexane; des hydrocarbures cycloaliphatiques tels que le cyclohexane; des hydrocarbures aromatiques tels que le benzène, le toluène, les xylènes; des hydrocarbures aromatiques chlorés tels que le monochlorobenzène et les polychlorobenzènes.

Parmi ces solvants le monochlorobenzène et les polychlorobenzènes sont particulièrement intéressants.

Essentiellement en raison de leurs points d'ébullition, on utilise plus préférentiellement les dichlorobenzènes et les trichlorobenzènes, lorsque le milieu solvant de la réaction est constitué par un mélange d'eau et de solvant organique.

Le rapport volumique eau/solvant organique n'est pas critique; l'eau peut représenter par exemple de 5% à 100% du volume total du milieu solvant. Le plus souvent l'eau représente de 50 à 100% du volume total du milieu solvant.

Dans ce qui suivra on emploiera par commodité les termes solution aqueuse pour désigner le milieu solvant dans lequel s'effectue la réaction, mais il est bien entendu que ces termes recouvrent également les solutions à base de mélanges eau/solvant organique tels que définies précédemment. Les concentrations des différents composés seront exprimées non par rapport à l'eau seule, mais par rapport au volume global du milieu solvant.

L'acidité du milieu peut varier dans de larges limites. De préférence la concentration en protons de la solution aqueuse est d'au moins 0,5 ions $H^+$ par litre. Il n'y a pas de valeur supérieure critique de cette concentration, bien qu'une trop grande acidité du milieu réactionnel ne soit pas désirée afin de limiter la corrosion de l'appareillage. En général la concentration en proton ne dépasse pas 15 ions $H^+$ par litre et, de préférence 8 ions $H^+$ par litre.

Le milieu aqueux acide dans lequel est conduite l'hydrogénation est constitué d'une solution aqueuse d'un acide minéral fort tel que les acides sulfurique et phosphorique et les hydracides et notamment les acides chlorhydrique, bromhydrique et iodhydrique. Ces derniers sont utilisés préférentiellement car on a constaté que la présence d'ions halogénures exerce une influence favorable sur le déroulement de la réaction. Pratiquement on a recours à des solutions aqueuses d'acide chlorhydrique puisque ce dernier se forme au cours de l'hydrodéchloration. On pourrait cependant, sans sortir du cadre de la présente invention, débuter l'hydrogénation en absence d'acide chlorhydrique, ce dernier étant apporté au milieu réactionnel par la réaction d'hydrodéchloration. On pourrait encore mettre en œuvre, comme milieu aqueux acide contenant des ions halogénures, une solution aqueuse d'un acide minéral fort halogéné ou non, et d'au moins un composé libérant des ions halogénures, de préférence chlorure, tel que les halogénures alcalins et alcalino-terreux, les

halogénures d'ammonium, les halogénures d'ammonium quaternaires, les halohydrates d'amines. En pareil cas, il est préférable d'avoir recours aux chlorures alcalins (chlorure de sodium et de potassium en particulier). La concentration en ions halogénures peut varier dans de larges limites en fonction de la nature de l'halogène. Ainsi lorsque l'halogénure présent dans le milieu réactionnel est l'iodure la concentration en ions $I^-$ peut être aussi faible que $1 \cdot 10^{-4}$ ion $I^-$/litre et de préférence au moins $1 \cdot 10^{-2}$ ion $I^-$/litre; lorsque l'halogénure est le bromure la concentration en ions bromure est d'au moins $1 \cdot 10^{-2}$ ion $Br^-$ et de préférence au moins 0,1 ion $Br^-$ par litre. Dans le cas où l'ion halogénure est l'ion chlorure, la concentration en ions $Cl^-$ par litre est d'au moins 2 et de préférence au moins 4. Quel que soit l'halogénure présent dans le milieu réactionnel, il n'y a pas de valeur maximale critique de la concentration en ions halogénures; cependant pour des raisons pratiques, il n'est pas nécessaire d'aller au delà d'une concentration de 15 et de préférence 8 ions-g/l. Lorsque l'on fait appel à des hydracides comme composés fournisseurs d'ions halogénures, il peut se révéler nécessaire de fournir un complément d'acidité quand la concentration en hydrocide est insuffisante pour amener la concentration en protons de la solution aqueuse dans les limites précitées. Dans ce cas on utilise conjointement un acide minéral fort tel que l'acide sulfurique. Comme cela a été exposé ci-avant le procédé selon l'invention est de préférence conduit au sein de solutions aqueuse d'acide chlorhydrique de concentration au moins égale à 2 moles d'HCl par litre; plus particulièrement la concentration des solutions aqueuses d'acide chlorhydrique est comprise entre 2 et 8 moles d'HCl par litre.

Comme métaux lourds convenant à la mise en œuvre du procédé selon la présente invention, on peut citer en particulier le bismuth, le plomb, l'étain, le thallium, le mercure et l'argent. L'argent et l'étain conviennent tout particulièrement bien.

Les métaux lourds peuvent être utilisés sous forme métallique ou sous forme de dérivés solubles dans la phase aqueuse acide et dans lesquels le reste associé au métal n'est pas critique. Ainsi on peut utiliser des oxydes, des sels d'acides minéraux ou organiques ou des chélates ou complexes métalliques. En pratique on fait plus particulièrement appel aux sels d'acides minéraux et notamment aux halogénures. A titre non limitatif, on peut citer parmi les sels métalliques utilisables: le chlorure stanneux, le chlorure stannique, le nitrate d'argent, le chlorure thalleux, le chlorure thallique, le sulfate de bismuth, le sulfate de plomb. En raison du caractère réducteur du milieu réactionnel, il s'est révélé préférable d'utiliser les métaux lourds sous forme métallique. A cet effet on emploie un catalyseur obtenu par coprécipitation d'un métal noble et d'au moins un des métaux lourds précités par réduction par les moyens usuels d'une solution aqueuse des sels correspondants, de préférence en présence d'un support.

La quantité de métal lourd mise en œuvre peut varier dans de large limites selon la forme sous laquelle il est utilisé. Lorsque le métal lourd est présent sous forme métallique dans le catalyseur à base de métal noble la quantité en est calculée pour que le rapport du nombre d'atomes-gramme de métal lourd au nombre d'atomes-gramme de métal noble soit d'au moins 0,3. Il n'y a pas de limite supérieure critique de la valeur de ce rapport mais il n'est pas nécessaire qu'il dépasse 10. De préférence ce rapport est compris entre 0,6 et 2. Quand le métal lourd est utilisé sous forme d'un dérivé soluble dans la phase aqueuse acide la quantité de ce dérivé doit être suffisante pour que la quantité de métal disponible au niveau des sites actifs du catalyseur à base de métal noble assure un bon déroulement de la réaction. Dans ce cas cette quantité est calculée de façon à ce que la concentration en cations métalliques de la phase aqueuse acide soit d'au moins $1 \cdot 10^{-4}$ ion-g/l de métal lourd et de préférence au moins $1 \cdot 10^{-2}$ ion-g/l. Il n'y a pas de valeur limite supérieure critique de cette concentration, mais pour des raisons pratiques évidentes il n'y a pas lieu de dépasser la limite de solubilité du dérivé métallique.

Les métaux nobles constituant la base des catalyseurs utilisés dans l'invention sont principalement des métaux du groupe VIII de la classification périodique tels que le ruthénium, le rhodium, le palladium, l'osmium, l'iridium et le platine; le palladium est le métal préféré. Le métal peut être à l'état métallique ou à l'état de composé chimique; généralement on préfère que le métal soit mis en œuvre à l'état métallique car, dans les conditions opératoires, les composés ont tendance à être réduit à l'état métallique. Le catalyseur peut être supporté ou non supporté. Comme support du catalyseur, on peut utiliser tout support connu en soi pourvu qu'il soit résistant à l'eau et aux acides; comme support convenant plus particulièrement, on peut citer le noir de carbone, la silice, le sulfate de baryum; le noir de carbone est un support préféré. Le catalyseur ainsi que son support sont avantageusement sous forme finement divisée; des surfaces spécifiques supérieures à 100 $m^2/g$ conviennent généralement bien.

La quantité de catalyseur mise en œuvre est telle que la proportion pondérale de métal noble du catalyseur par rapport au composé de formule (I) à traiter est généralement comprise entre 0,01 et 10%, de préférence entre 0,1 et 5%.

La température de la réaction est généralement comprise entre 50 et 350°C et, de préférence entre 100 et 250°C.

La pression partielle d'hydrogène peut varier dans de larges limites et être supérieure, inférieure ou égale à la pression atmosphérique. Plus spécifiquement la pression d'hydrogène est comprise entre 0,1 et 60 bars et, de préférence entre 0,5 et 50 bars. On pourrait avoir reccours à des pressions

supérieures à 60 bars mais sans que cela se traduise par des avantages particuliers. La pression totale à laquelle s'effectue la réaction dépend essentiellement des conditions de température, de la volatilité de l'acide mis en œuvre dans ces conditions et de la valeur de la pression partielle d'hydrogène. Il va de soi que la pression totale doit être suffisante pour maintenir le milieu réactionnel liquide et/ou la concentration en acide de la phase aqueuse dans les limites précitées.

Comme exemples de polychlorophénols de formule (I) qui peuvent être utilisés comme matières premières dans le procédé conforme à la présente invention, on peut citer:

- le dichloro-2,3 phénol
- le dichloro-2,5 phénol
- le dichloro-3,4 phénol
- le trichloro-2,3,4 phénol
- le trichloro-2,3,6 phénol
- le trichloro-2,4,5 phénol
- le trichloro-2,3,5 phénol
- le trichloro-3,4,5 phénol
- le tétrachloro-2,3,4,6 phénol
- le tétrachloro-2,3,4,5 phénol
- le tétrachloro-2,3,5,6 phénol
- le pentachlorophénol
- le trichloro-2,3,4 méthyl-6 phénol
- le dichloro-2,3 méthyl-6 phénol
- le tétrachloro-2,3,4,6 méthyl-5 phènol
- le dichloro-2,3 méthyl-4 phénol
- le tétrachloro-2,3,5,6 méthyl-4 phénol
- le dichloro-2,5 diméthyl-3,4 phénol
- le dichloro-2,5 éthyl-4 phénol
- le dichloro-2,5 propyl-4 phénol
- le dichloro-2,5 t-butyl-4 phénol
- le trichloro-3,4,6 benzyl-2 phénol
- le dichloro-3,4 méthoxy-2 phénol
- le dichloro-3,6 méthoxy-2 phénol
- le dichloro-4,5 méthoxy-2 phénol
- le dichloro-5,6 méthoxy-2 phénol
- le trichloro-3,4,6 méthoxy-2 phénol
- le trichloro-3,4,5 méthoxy-2 phénol
- le tétrachloro-3,4,5,6 méthoxy-2 phénol
- le dichloro-4,5 méthoxy-3 phénol
- le dichloro-5,6 méthoxy-3 phénol
- le dichloro-2,5 méthoxy-3 phénol
- le trichloro-4,5,6 méthoxy-3 phénol
- le trichloro-2,3,6 méthoxy-3 phénol
- le dichloro-4,5 phénoxy-2 phénol
- le tétrachloro-2,3,5,6 phénoxy-4 phénol
- le dichloro-3,4 éthoxy-2 phénol
- le trichloro-3,4,5 éthoxy-2 phénol
- le dichloro-3,4 phényl-2 phénol
- le trichloro-3,5,6 phényl-2 phénol.

En pratique on fait appel de préférence aux di- et trichlorophénols.

Parmi les phénols comportant un atome de chlore sur l'une au moins des positions en méta par rapport à l'hydroxyle phénolique qui peuvent être préparés par le procédé selon la présente invention, on peut citer:

- le chloro-3 phénol
- le dichloro-3,5 phénol
- le chloro-3 méthyl-6 phénol
- le chloro-3- méthyl-5-phénol
- le chloro-3 méthyl-4 phénol
- le dichloro-3,5 méthyl-4 phénol
- le chloro-5 diméthyl-3,4 phénol
- le dichloro-3,5 éthyl-4 phénol
- le dichloro-3,5 propyl-4 phénol
- le dichloro-3,5 t-butyl-4 phénol
- le chloro-3 benzyl-2 phénol

- le chloro-3 méthoxy-2 phénol
- le chloro-3 méthoxy-6 phénol
- le dichloro-3,5 méthoxy-2 phénol
- le chloro-3 méthoxy-5 phénol
- le chloro-3 phénoxy-6 phénol
- le dichloro-3,5 phénoxy-6 phénol
- le chloro-3 éthoxy-2 phénol
- le chloro-3-phényl-2-phénol.

Le procédé selon l'invention peut être mis en œuvre de façon continue ou discontinue. En fin de réaction le catalyseur est séparé par filtration et peut être recyclé tel quel dans une nouvelle opération d'hydrodéchloration. Les métachlorophénols formés peuvent être séparés du milieu réactionnel par extraction à l'aide d'un solvant organique non miscible à l'eau puis récupérés par distillation après élimination du solvant d'extraction.

Les exemples suivants illustrent l'invention et montrent comment elle peut être mise en pratique.

## Exemple 1

Dans un autoclave de 250 ml en acier inoxydable comportant un revêtement intérieur en tantale, équipé d'un système d'agitation, on charge:

- 1 g de dichloro-3,4 phénol
- 100 ml d'une solution aqueuse d'acide chlorhydrique 6 N
- 0,210 g d'un catalyseur au palladium déposé sur un charbon actif de surface spécifique 1000 $m^2 \cdot g^{-1}$ contenant 2% en poids de palladium métal (soit 4,2 mg de palladium) et 3% en poids d'argent métal (soit 6,3 mg d'argent).

On porte le contenu de l'autoclave à 190°C après avoir fermé ce dernier, puis on introduit de l'hydrogène jusqu'à ce que la pression totale atteigne 45 bars et maintient ces conditions pendant 5 heures et 50 minutes. On refroidit ensuite le contenu de l'autoclave, le dégaze puis le soutire. On sépare ensuite le catalyseur de la phase aqueuse. Les chlorophénols sont extraits de la phase aqueuse par 300 ml d'éther. Le catalyseur est lavé par 3 fois 20 ml d'éther pour en extraire les chlorophénols qu'il contient. Un réunit les extraits éthérés puis élimine l'éther par distillation et dose et identifie les chlorophénols présents dans le résidu de distillation par chromatographie en phase vapeur.

Les résulats de l'analyse montrent que la totalité du dichloro-3,4 phénol a été transformée [taux de transformation (TT): 100%]. On a identifié dans le résidu de distillation:

- du chloro-3 phénol: rendement par rapport au dichloro-3,4 phénol chargé (RR) = 72%
- du phénol: RR = 28%.

## Exemple 2

On a reproduit l'exemple 1 mais après avoir remplacé le catalyseur Pd/Ag par 0,14 g d'un catalyseur au palladium sur noir (5% en poids de Pd déposé sur noir de carbone de surface spécifique 1000 $m^2 \cdot g^{-1}$) et en opérant en présence de 0,19 g de chlorure stanneux.

Aprés 6 h de réaction à 190°C sous une pression totale de 43 bars, on refroidit le contenu de l'autoclave et le traite comme à l'exemple 1. On a obtenu les résultats suivants:

| | | | |
|---|---|---|---|
| — | TT | dichloro-3,4 phénol | 100% |
| — | RR | chloro-3 phénol | 68% |
| — | RR | chloro-4 phénol | 2,5% |
| — | RR | phenol | 29,5% |

## Exemple 3

On reproduit l'exemple 1 avec les charges suivantes:

- 1 g de dichloro-2,5 phénol
- 0,3 g de Pd/charbon à 2% de palladium métal et 3% d'argent métal (identique à celui de l'exemple 1)
- 100 ml d'acide chlorhydrique aqueux 6 N

On fait réagir dans les conditions de l'exemple 1 pendant 5 heures.

Après le même traitement que dans l'exemple 1, on obtient les résultats suivants:

- TT du dichloro-2,5 phénol 87%
- RT en chloro-5 phénol 69%
- RT en chloro-2 phénol 14%
- RT en phénol 17%

Exemple 4

On reproduit l'exemple 1 avec les charges suivantes:

- 1,6 g de pentachlorophénol ($6 \times 10^{-3}$ moles)
- 0,3 g de Pd/charbon à 2% de palladium métal et 3% d'argent métal (identique à celui de l'exemple 1)
- 100 ml d'acide chlorhydrique aqueux 6 N

Après 40 heures de réaction à 210°C sous une pression totale fe 65 bars, on refroidit le contenu de l'autoclave et on le traite comme dans l'exemple 1.

On obtient les résultats suivants:

- TT du pentachlorophénol 100%
- RR en dichloro-3,5 phénol 83%
- RR en tétrachloro-2,3,5,6 phénol 2,9%
- RR en trichloro-2,3,5 phénol 4,8%
- RR en chloro-3 phénol 9,3%

**Revendications**

1. Procédé d'obtention sélective de chlorophénols comportant un atome de chlore sur l'une au moins des positions méta par rapport à l'hydroxyle phénolique, par hydrogénation à chaud en phase liquide, en présence d'un catalyseur à base d'un métal noble du groupe VIII de la classification périodique, de polychlorophénols de formule générale (I):

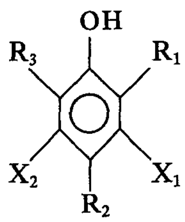

(I)

dans laquelle:

- $X_1$ et $X_2$, identiques ou différents représentent un atome de chlore, un atome d'hydrogène, un radical alkyle, aryle, arylalkyle, alkyloxy, aryloxy, l'un au moins des symboles $X_1$ et $X_2$ représentant un atome de chlore,
- $R_1$, $R_2$ et $R_3$, identiques ou différents, représentent un atome de chlore, un atome d'hydrogène, un radical alkyle, un radical aryle, arylalkyle, un radical alkyloxy, aryloxy, l'un au moins des symboles $R_1$, $R_2$ et $R_3$ représentant un atome de chlore,

caractérisé en ce que la réaction est conduite dans un milieu solvant acide au moins partiellement aqueux, en présence d'au moins un métal lourd appartenant aux groupes 1b, 2b, 3a, 4a et 5a de la classification périodique des éléments.

2. Procédé selon la revendication 1, caractérisé en ce que le milieu solvant au moins partiellement aqueux est constitué par de l'eau ou par un mélange eau/solvant organique comportant au moins 5% en volume d'eau et de préférence au moins 50% en volume d'eau.

3. Procédé selon l'une des revendications 1 ou 2, caractérisé en ce que ceux des radicaux $X_1$, $X_2$, $R_1$, $R_2$ et $R_3$ qui ne symbolisent pas un atome de chlore représentent un radical alkyle comportant de 1 à 10 atomes de carbone; un radical phényle; un radical benzyle; un radical alkyloxy comportant de 1 à 10 atomes de carbone; un radical phénoxy.

4. Procédé selon l'une quelconque des revendications 1 à 3, caractérisé en ce que la concentration en protons de la solution aqueuse acide est d'au moins 0,5 ion-g/l.

5. Procédé selon l'une quelconque des revendications 1 à 4, caractérisé en ce que la concentration en protons de la solution aqueuse acide est au plus égale à 15 ions-g/l.

6. Procédé selon l'une quelconque des revendications 1 à 5, caractérisé en ce que le milieu acide contient des ions halogénures.

7. Procédé selon la revendication 6, caractérisé en ce que la concentration en ions halogénures de la solution aqueuse acide est d'au moins $1 \cdot 10^{-4}$ ion-g/l.

8. Procédé selon la revendication 6, caractérisé en ce que la concentration en ions halogénures de la solution aqueuse acide est au plus égale à 15 ion-g/l.

9. Procédé selon la revendication 6, caractérisé en ce que le milieu acide contient des ions chlorure.

10. Procédé selon l'une quelconque des revendications 1 à 9, caractérisé en ce que le milieu aqueux acide est constitué par une solution aqueuse d'acide chlorhydrique.

11. Procédé selon la revendication 10, caractérisé en ce que la solution aqueuse d'acide chlorhydrique a une concentration comprise entre 2 moles $\cdot\ 1^{-1}$ et 8 moles $\cdot\ 1^{-1}$.

12. Procédé selon l'une quelconque des revendications 1 à 11, caractérisé en ce que le métal lourd est sous forme métallique.

13. Procédé selon l'une quelconque des revendications 1 à 11, caractérisé en ce que le métal lourd est sous forme d'un composé minéral ou organique soluble dans le milieu aqueux acide.

14. Procédé selon la revendication 13, caractérisé en ce que le métal lourd est sous forme d'un chlorure.

15. Procédé selon l'une quelconque des revendications 1 à 14, caractérisé en ce que le métal lourd est pris dans le groupe formé par Bi, Pb, Sn, Tl, Hg et Ag.

16. Procédé selon la revendication 12, caractérisé en ce que le métal est l'argent.

17. Procédé selon la revendication 13, caractérisé en ce que le dérivé métallique est le chlorure stanneux.

18. Procédé selon l'une des revendications 1 à 17, caractérisé en ce que le catalyseur est du palladium déposé sur un support inerte.

19. Procédé selon la revendication 18, caractérisé en ce que la quantité de catalyseur exprimée en poids de métal noble pour 100 g de polychlorophénol de formule (I) est comprise entre 0,01 g et 10 g.

20. Procédé selon la revendication 12, caractérisé en ce que la quantité de métal lourd est calculée pour le rapport du nombre d'atomes-gramme de ce métal au nombre d'atomes-gramme de métal noble soit d'au moins 0,3.

21. Procédé selon la revendication 13, caractérisé en ce que la quantité de dérivé de métal lourd est calculée pour la concentration de la solution aqueuse acide en ions de métal lourd soit d'au moins $1 \cdot 10^{-4}$ ion-g par litre.

22. Procédé selon l'une quelconque des revendications 1 à 21, caractérisé en ce que la température de la réaction est comprise entre 50 et 350° C.

23. Procédé selon l'une quelconque des revendications 1 à 22, caractérisé en ce que la pression partielle d'hydrogène est comprise entre 0,1 et 60 bars.

24. Procédé selon l'une quelconque des revendications 1 à 23, caractérisé en ce que le polychlorophénol de formule (I) est pris dans le groupe formé par les dichloro- et trichlorophénols comportant un atome de chlore sur l'une au moins des positions méta par rapport à l'hydroxyle phénolique.

25. Procédé selon l'une des revendications 1 à 23, caractérisé en ce que le polychlorophénol de formule (I) est pris dans le groupe formé par le dichloro-3,4 phénol, le dichloro-2,5 phénol et le pentachlorophénol.

## Patentansprüche

1. Verfahren zur selektiven Erzielung von Chlorphenolen mit einem Chloratom in wenigstens einer der meta-Stellungen im Bezug auf das phenolische Hydroxyl durch Hydrierung in der Wärme in flüssiger Phase in Gegenwart eines Katalysators auf Basis eines Edelmetalls der Gruppe VIII des Periodensystems, von Chlorphenolen der allgemeinen Formel (I)

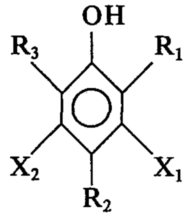

(I)

worin:

— $X_1$ und $X_2$, die identisch oder verschieden sind, bedeuten ein Chloratom, ein Wasserstoffatom,

einen Alkyl-, Aryl-, Arylalkyl-, Alkyloxy-, Aryloxyrest, wobei wenigstens eines der Symbole $X_1$ und $X_2$ ein Chloratom bedeutet,

— $R_1$, $R_2$ und $R_3$, die identisch oder verschieden sind, bedeuten ein Chloratom, ein Wasserstoffatom, einen Alkylrest, einen Aryl-, Arylalkylrest, einen Alkyloxy-, Aryloxyrest, wobei wenigstens eines der Symbole $R_1$, $R_2$ und $R_3$ ein Chloratom bedeutet,

dadurch gekennzeichnet, daß die Reaktion in einem sauren, wenigstens teilweise wässerigen Lösungsmittelmilieu in Gegenwart von wenigstens einem Schwermetall aus der Gruppe 1b, 2b, 3a, 4a und 5a des Periodensystems durchgeführt wird.

2. Verfahren gemäß Anspruch 1, dadurch gekennzeichnet, daß das wenigstens teilweise wässerige Lösungsmittelmilieu aus Wasser oder aus einem Gemisch Wasser/organisches Lösungsmittel mit wenigstens 5 Vol.-% Wasser und vorzugsweise wenigstens 50 Vol.-% Wasser besteht.

3. Verfahren gemäß einem der Ansprüche 1 oder 2, dadurch gekennzeichnet, daß solche Reste $X_1$, $X_2$, $R_1$, $R_2$ und $R_3$, die kein Chloratom bedeuten, einen Alkylrest mit 1 bis 10 Kohlenstoffatomen; einen Phenylrest; einen Benzylrest; einen Alkyloxyrest mit 1 bis 10 Kohlenstoffatomen; einen Phenoxyrest bedeuten.

4. Verfahren gemäß einem der Ansprüche 1 bis 3, dadurch gekennzeichnet, daß die Konzentration der wässerigen sauren Lösung an Protonen wenigstens 0,5 Ion-g/l beträgt.

5. Verfahren gemäß einem der Ansprüche 1 bis 4, dadurch gekennzeichnet, daß die Konzentration der sauren wässerigen Lösung an Protonen höchstens gleich 15 Ionen-g/l beträgt.

6. Verfahren gemäß einem der Ansprüche 1 bis 5, dadurch gekennzeichnet, daß das saure Milieu Halogenidionen enthält.

7. Verfahren gemäß Anspruch 6, dadurch gekennzeichnet, daß die Konzentration der sauren wässerigen Lösung an Halogenidionen mindestens $1 \cdot 10^{-4}$ Ion-g/l beträgt.

8. Verfahren gemäß Anspruch 6, dadurch gekennzeichnet, daß die Konzentration der sauren wässerigen Lösung an Halogenidionen höchstens gleich 15 Ionen-g/l beträgt.

9. Verfahren gemäß Anspruch 6, dadurch gekennzeichnet, daß das saure Milieu Chloridionen enthält.

10. Verfahren gemäß einem der Ansprüche 1 bis 9, dadurch gekennzeichnet, daß das saure wässerige Milieu aus einer wässerigen Lösung von Chlorwasserstoffsäure besteht.

11. Verfahren gemäß Anspruch 10, dadurch gekennzeichnet, daß die wässerige Lösung von Chlorwasserstoffsäure eine Konzentration zwischen 2 Mol $\cdot$ $1^{-1}$ und 8 Mol $\cdot$ $1^{-1}$ beträgt.

12. Verfahren gemäß einem der Ansprüche 1 bis 11, dadurch gekennzeichnet, daß das Schwermetall in metallischer Form vorliegt.

13. Verfahren gemäß einem der Ansprüche 1 bis 11, dadurch gekennzeichnet, daß das Schwermetall in Form einer mineralischen oder organischen Verbindung, welche in dem sauren wässerigen Milieu löslich ist, vorliegt.

14. Verfahren gemäß Anspruch 13, dadurch gekennzeichnet, daß das Schwermetall in Form eines Chlorids vorliegt.

15. Verfahren gemäß einem der Ansprüche 1 bis 14, dadurch gekennzeichnet, daß das Schwermetall genommen ist aus der Gruppe, gebildet von Bi, Pb, Sn, Tl, Hg und Ag.

16. Verfahren gemäß Anspruch 12, dadurch gekennzeichnet, daß das Metall Silber ist.

17. Verfahren gemäß Anspruch 13, dadurch gekennzeichnet, daß das Metallderivat Zinn-II-Chlorid ist.

18. Verfahren gemäß einem der Ansprüche 1 bis 17, dadurch gekennzeichnet, daß der Katalysator Palladium, abgelagert auf einem inerten Träger, ist.

19. Verfahren gemäß Anspruch 18, dadurch gekennzeichnet, daß die Katalysatormenge, ausgedrückt in Gewicht Edelmetall für 100 g Polychlorphenol der Formel (I), zwischen 0,01 g und 10 g liegt.

20. Verfahren gemäß Anspruch 12, dadurch gekennzeichnet, daß die Menge Schwermetall derart berechnet ist, daß das Verhältnis der Anzahl Gramm-Atome dieses Metalls zur Anzahl Gramm-Atome des Edelmetalls mindestens 0,3 beträgt.

21. Verfahren gemäß Anspruch 13, dadurch gekennzeichnet, daß die Menge an Schwermetallderivat derart berechnet ist, daß die Konzentration der sauren wässerigen Lösung an Schwermetallionen mindestens $1 \cdot 10^{-4}$ Ion-g/l ist.

22. Verfahren gemäß einem der Ansprüche 1 bis 21, dadurch gekennzeichnet, daß die Reaktionstemperatur zwischen 50 und 350° C beträgt.

23. Verfahren gemäß einem der Ansprüche 1 bis 22, dadurch gekennzeichnet, daß der Wasserstoffpartialdruck zwischen 0,1 und 60 bar beträgt.

24. Verfahren gemäß einem der Ansprüche 1 bis 23, dadurch gekennzeichnet, daß das Polychlorphenol der Formel (I) aus der Gruppe genommen ist, gebildet von den Dichlor- und Trichlorphenolen mit einem Chloratom an wenigstens einer der meta-Stellungen in bezug auf das phenolische Hydroxyl.

25. Verfahren gemäß einem der Ansprüche 1 bis 23, dadurch gekennzeichnet, daß das Polychlorphenol der Formel (I) genommen ist aus der Gruppe, gebildet von 3,4-Dichlorphenol,

2,5-Dichlorphenol und Pentachlorphenol.

**Claims**

1. Process for selectively obtaining chlorophenols containing a chlorine atom in at least one of the metapositions relative to the phenolic hydroxyl, by the hydrogenation, under the action of heat, in the liquid phase, in the presence of a catalyst based on a noble metal of group VIII of the periodic classification, of polychlorophenols of the general formula (I):

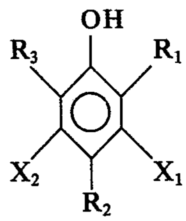

(I)

in which:

— $X_1$ and $X_2$, which are identical or different, represent a chlorine atom, a hydrogen atom or an alkyl, aryl, arylalkyl, alkoxy or aryloxy radical, at least one of the symbols $X_1$ and $X_2$ representing a chlorine atom, and

— $R_1$, $R_2$ and $R_3$, which are identical or different, represent a chlorine atom, a hydrogen atom, an alkyl radical, an aryl or arylalkyl radical or an alkoxy or aryloxy radical, at least one of the symbols $R_1$, $R_2$ and $R_3$ representing a chlorine atom,

characterised in that the reaction is carried out in an acid solvent medium which is at least partially aqueous, in the presence of at least one heavy metal belonging to groups 1b, 2b, 3a, 4a or 5a of the periodic classification of the elements.

2. Process according to Claim 1, characterised in that the at least partially aqueous solvent medium consists of water or of a water/organic solvent mixture containing at least 5% by volume of water and preferably at least 50% by volume of water.

3. Process according to one of Claims 1 or 2, characterised in that those radicals $X_1$, $X_2$, $R_1$, $R_2$ and $R_3$ which do not symbolise a chlorine atom represent an alkyl radical containing from 1 to 10 carbon atoms, a phenyl radical, a benzyl radical, an alkoxy radical containing from 1 to 10 carbon atoms or a phenoxy radical.

4. Process according to any one of Claims 1 to 3, characterised in that the concentration of protons in the acid aqueous solution is at least 0.5 g ion/litre.

5. Process according to any one of Claims 1 to 4, characterised in that the concentration of protons in the acid aqueous solution is equal to at most 15 g ions/litre.

6. Process according to any one of Claims 1 to 5, characterised in that the acid medium contains halide ions.

7. Process according to Claim 6, characterised in that the concentration of halide ions in the acid aqueous solution is at least $1 \cdot 10^{-4}$ g ion/litre.

8. Process according to Claim 6, characterised in that the concentration of halide ions in the acid aqueous solution is equal to at most 15 g ions/litre.

9. Process according to Claim 6, characterised in that the acid medium contains chloride ions.

10. Process according to any one of Claims 1 to 9, characterised in that the acid aqueous medium consists of an aqueous solution of hydrochloric acid.

11. Process according to Claim 10, characterised in that the aqueous solution of hydrochloric acid has a concentration of between 2 mols $\cdot$ litre$^{-1}$ and 8 Mols $\cdot$ litre$^{-1}$.

12. Process according to any one of Claims 1 to 11, characterised in that the heavy metal is in the metallic form.

13. Process according to any one of Claims 1 to 11, characterised in that the heavy metal is in the form of an inorganic or organic compound which is soluble in the acid aqueous medium.

14. Process according to Claim 13, characterised in that the heavy metal is in the form of a chloride.

15. Process according to any one of Claims 1 to 14, characterised in that the heavy metal is taken from the group comprising Bi, Pb, Sn, Tl, Hg and Ag.

16. Process according to Claim 12, characterised in that the metal is silver.

17. Process according to Claim 13, characterised in that the metal derivative is stannous chloride.

18. Process according to one of Claims 1 to 17, characterised in that the catalyst is palladium deposited on an inert support.

19. Process according to Claim 18, characterised in that the amount of catalyst, expressed as the weight of noble metal per 100 g of polychlorophenol of the formula (I), is between 0.01 g and 10 g.

20. Process according to Claim 12, characterised in that the amount of heavy metal is calculated so that the ratio of the number of gram atoms of this metal to the number of gram atoms of noble metal is at least 0.3.

21. Process according to Claim 13, characterised in that the amount of heavy metal derivative is calculated so that the concentration of heavy metal ions in the acid aqueous solution is at least $1 \cdot 10^{-4}$ g ion per litre.

22. Process according to any one of Claims 1 to 21, characterised in that the reaction temperature is between 50 and 350° C.

23. Process according to any one of Claims 1 to 22, characterised in that the hydrogen partial pressure is between 0.1 and 60 bars.

24. Process according to any one of claims 1 to 23, characterised in that the polychlorophenol of the formula (I) is taken from the group comprising the dichlorophenols and trichlorophenols containing a chlorine atom in at least one of the meta-positions relative to the phenolic hydroxyl.

25. Process according to Claim 24, characterised in that the polychlorophenol of the formula (I) is taken from the group comprising 3,4-dichlorophenol, 2,5-dichlorophenol and pentachlorophenol.